(19) Europäisches Patentamt
European Patent Office
Office européen des brevets

(11) **EP 3 251 663 A1**

(12) **EUROPEAN PATENT APPLICATION**
published in accordance with Art. 153(4) EPC

(43) Date of publication:
**06.12.2017 Bulletin 2017/49**

(21) Application number: **16743408.3**

(22) Date of filing: **27.01.2016**

(51) Int Cl.:
*A61K 9/70* (2006.01)  *A61K 31/137* (2006.01)
*A61K 31/27* (2006.01)  *A61K 31/618* (2006.01)
*A61K 47/06* (2006.01)  *A61K 47/10* (2017.01)
*A61K 47/14* (2017.01)  *A61K 47/32* (2006.01)
*A61K 47/38* (2006.01)  *A61P 17/00* (2006.01)
*A61P 25/16* (2006.01)  *A61P 25/28* (2006.01)

(86) International application number:
**PCT/JP2016/052294**

(87) International publication number:
**WO 2016/121805 (04.08.2016 Gazette 2016/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **30.01.2015 JP 2015016991**

(71) Applicant: **TOYO INK SC HOLDINGS CO., LTD.**
**Chuo-ku**
**Tokyo 104-0031 (JP)**

(72) Inventors:
• **KOMODA, Toshikazu**
 **Amagasaki-shi**
 **Hyogo 661-8564 (JP)**
• **OBATA, Kumi**
 **Amagasaki-shi**
 **Hyogo 661-8564 (JP)**

(74) Representative: **Ter Meer Steinmeister & Partner**
**Patentanwälte mbB**
**Nymphenburger Straße 4**
**80335 München (DE)**

(54) **ADHESIVE PATCH**

(57)    Provided is an adhesive patch capable of exerting excellent tackiness when the adhesive patch is attached to the skin and having a low incidence of detachment from the skin during attachment. The adhesive patch of the present invention includes a support, and an adhesive layer integrally layered on one surface of the support, the adhesive layer containing an acrylic adhesive, a solid resin that is incompatible with the acrylic adhesive and is in a solid state at 40°C, a plasticizer, and a drug. The combined use of the solid resin and the plasticizer can simultaneously achieve the tackiness and cohesive force of the adhesive layer.

EP 3 251 663 A1

**Description**

Technical Field

The present invention relates to an adhesive patch for transdermal administration of a drug.

Background Art

Conventionally, an adhesive patch has been used to allow a drug to be transdermally absorbed into the body. The adhesive patch generally includes a support, and an adhesive layer containing an adhesive and a drug. As the adhesive, an acrylic adhesive, a rubber-based adhesive, a silicone-based adhesive, or the like is used. Among these, the acrylic adhesive is often used (for example, Patent Literature 1) since the acrylic adhesive allows function and transdermal absorption performance to be selected according to combination with a monomer.

Citation List

Patent Literature

Patent Literature 1: Japanese Patent No. 4724368

Summary of Invention

Technical Problem

An adhesive layer containing an acrylic adhesive can exert sufficient tackiness when an adhesive patch is attached to the skin. However, when a long time elapses after the attachment, the tackiness of the adhesive layer may be reduced, to detach the adhesive patch from the skin.

Then, the addition of a plasticizer to the adhesive layer can maintain the tackiness over a long time. However, it is necessary that the adhesive layer contain a predetermined amount of a drug to impart a drug effect to the adhesive layer. As a result, the thickness of the adhesive layer increases. The addition of the plasticizer to such a thick adhesive layer may destroy the inside of the adhesive layer and split up the adhesive layer by repeated motion of the human body, so that the adhesive patch may be detached during attachment.

Therefore, the improvement in the cohesive force of the adhesive layer can reduce the destruction of the adhesive layer by repeated motion of the human body. However, the improvement in the cohesive force of the adhesive layer may reduce the tackiness of the adhesive layer, and the adhesive patch may be hardly attached when the adhesive patch is attached to the skin. Therefore, the tackiness and cohesive force of the adhesive layer have contrary relation, and it is difficult to simultaneously achieve both the tackiness and cohesive force.

An object of the present invention is to provide an adhesive patch capable of exerting excellent tackiness when the adhesive patch is attached to the skin and having a low incidence of detachment from the skin during attachment.

Means for Solving Problem

The adhesive patch of the present invention includes a support and an adhesive layer integrally layered on one surface of the support.

[Adhesive Layer]

The adhesive layer contains an acrylic adhesive, a solid resin that is incompatible with the acrylic adhesive and is in a solid state at 40°C, a plasticizer, and a drug.

The use of the plasticizer can plasticize the acrylic adhesive, and improve the anchoring properties of the adhesive layer to the skin surface. When the adhesive patch is attached, the adhesive layer can exert excellent tackiness to the skin. Therefore, the adhesive patch can be certainly attached to the skin. The use of the solid resin can enhance the cohesive force of the adhesive layer. Therefore, the destruction of the adhesive layer by repeated motion of the human body can be reduced, and the adhesive patch is capable of following the motion of the human body. Accordingly, detachment of the adhesive patch can be reduced during attachment of the adhesive patch to the skin. The combined use of the solid resin and the plasticizer can simultaneously achieve both the tackiness and cohesive force of the adhesive layer. Therefore, an adhesive patch capable of exerting excellent tackiness to the skin when the adhesive patch is attached and having a low incidence of detachment from the skin during attachment can be provided.

Further, the combined use of the solid resin and the plasticizer can reduce the resistance to a high-speed and large detachment force applied during detachment and removal of the adhesive patch after administration of the drug. The adhesive patch can be easily detached and removed so as not to cause skin stimulation.

(Acrylic Adhesive)

Examples of the acrylic adhesive include acrylic polymers obtained by polymerization of a monomer containing alkyl (meth)acrylate. Herein, (meth)acrylate means acrylate or methacrylate. The acrylic polymer may be used alone, or two or more kinds thereof may be used in combination.

The number of carbon atoms in the alkyl group of alkyl(meth)acrylate is preferably 1 to 16, more preferably 1 to 14, particularly preferably 2 to 14, and the most preferably 2 to 12.

Examples of alkyl (meth)acrylate having an alkyl group having 1 to 16 carbon atoms include methyl (meth)acrylate, ethyl (meth)acrylate, n-propyl (meth)acrylate, isopropyl (meth)acrylate, n-butyl (meth)acrylate, isobutyl (meth)acrylate, hexyl (meth)acrylate, n-octyl (meth)acrylate, isooctyl (meth)acrylate, 2-ethylhexyl (meth)acrylate, decyl (meth)acrylate, dodecyl (meth)acrylate, tridecyl (meth)acrylate, hexadecyl (meth)acrylate, cyclododecyl (meth)acrylate, cyclohexyl (meth)acrylate, hydroxyethyl (meth)acrylate, and hydroxypropyl (meth)acrylate. Among these, n-octyl (meth)acrylate and 2-ethylhexyl (meth)acrylate are preferable. The alkyl (meth)acrylate may be used alone, or two or more kinds thereof may be used in combination.

The acrylic polymer is preferably a copolymer of alkyl (meth)acrylate having an alkyl group having 1 to 16 carbon atoms including alkyl (meth)acrylate having an alkyl group having 3 to 16 carbon atoms.

Examples of alkyl (meth)acrylate having an alkyl group having 3 to 16 carbon atoms include n-propyl (meth)acrylate, n-butyl (meth)acrylate, hexyl (meth)acrylate, 2-ethylbutyl (meth)acrylate, isooctyl (meth)acrylate, 2-ethylhexyl (meth)acrylate, decyl (meth)acrylate, dodecyl (meth)acrylate, and tridecyl (meth)acrylate. Among these, dodecyl (meth)acrylate is preferable.

An acrylic copolymer is preferably an acrylic copolymer obtained by copolymerization of a monomer containing n-octyl (meth)acrylate, 2-ethylhexyl (meth)acrylate, and dodecyl (meth)acrylate, more preferably an acrylic copolymer obtained by copolymerization of a monomer containing 2-ethylhexyl (meth)acrylate and dodecyl (meth)acrylate, and particularly preferably an acrylic copolymer obtained by copolymerization of a monomer containing dodecyl methacrylate, 2-ethylhexyl acrylate, and 2-ethylhexyl methacrylate.

The content of alkyl (meth)acrylate component having an alkyl group having 1 to 16 carbon atoms in the acrylic copolymer is preferably 40% by weight or more, and more preferably 45 to 95% by weight.

The content of alkyl (meth)acrylate having an alkyl group having 3 to 16 carbon atoms in the acrylic copolymer is preferably less than 60% by weight, and more preferably 5 to 55% by weight.

In addition to alkyl (meth)acrylate, the monomer of the acrylic copolymer may include another monomer. Examples of the other monomer include 1-vinyl-2-pyrrolidone, acrylamide, dimethylacrylamide, acrylonitrile, dimethylaminoethyl (meth)acrylate, tert-butylaminoethyl (meth)acrylate, vinyl acetate, and vinyl propionate. Herein, (meth)acrylic acid means acrylic acid or methacrylic acid.

As a polymerization method for the acrylic polymer, a conventionally well-known method may be performed. Examples thereof include a method in which the aforementioned monomer is polymerized in the presence of a polymerization initiator. Specifically, a predetermined amount of the monomer, the polymerization initiator, a polymerization solvent, and if necessary, a crosslinking agent are supplied to a reaction vessel, and heated at 60 to 80°C for 4 to 48 hours, resulting in radical polymerization of the monomer.

Examples of the polymerization initiator include an azobis-based polymerization initiator, such as 2,2'-azobisisobutyronitrile (AIBN), 1,1'-azobis(cyclohexane-1-carbonitrile), and 2,2'-azobis(2,4'-dimethylvaleronitrile); and a peroxide-based polymerization initiator, such as benzoyl peroxide (BPO), lauroyl peroxide (LPO), and di-tert-butyl peroxide. Examples of the polymerization solvent include ethyl acetate and toluene. Further, it is preferable that the polymerization reaction be carried out under a nitrogen gas atmosphere.

The content of the acrylic adhesive in the adhesive layer is preferably 45 to 90% by weight, more preferably 50 to 85% by weight, and particularly preferably 55 to 80% by weight. The acrylic adhesive contained in a smaller amount may reduce the tackiness of the adhesive layer. The acrylic adhesive contained in a larger amount may hinder the addition of the drug and plasticizer to the adhesive layer in required amounts.

(Solid Resin)

The adhesive layer contains the solid resin that is incompatible with the acrylic adhesive described above and is in a solid state at 40°C. Herein, the solid state means a state having no fluidity.

The difference ($|SP_S - SP_A|$) between a solubility parameter ($SP_A$) of the acrylic adhesive and a solubility parameter ($SP_S$) of the solid resin is preferably 0.5 $(cal/cm^3)^{1/2}$ or more, more preferably 0.8 $(cal/cm^3)^{1/2}$ or more, particularly preferably 0.8 to 3 $(cal/cm^3)^{1/2}$, and the most preferably 1 to 3 $(cal/cm^3)^{1/2}$. Confining the difference between the solubility parameters to fall within the aforementioned range can provide a solid resin incompatible with the acrylic adhesive.

Since the solid resin is incompatible with the acrylic adhesive, the acrylic adhesive and the solid resin are each subjected to phase separation and are present as independent phases without mixing and forming a single phase.

The solubility parameter is the square root of density per unit volume of intermolecular cohesive energy, which is defined by J. H. Hildebrand. Specifically, the solubility parameter $\delta$ is determined by the following equation (1).

$$\delta = (E/V)^{1/2} \quad (1)$$

(wherein $\delta$ is a solubility parameter, E is molar cohesive energy, and V is a molar volume.)

As the solubility parameter, values described in "Youkaisei Parameter Tekiyo Jirei-syu (Case Studies of Application of Solubility Parameter)" (Akitoshi Taniguchi, Johokiko Co., Ltd., March 15, 2007, pp.276 to 282) and the like can be referred to.

The solubility parameter of the acrylic adhesive is the square root of the sum of values obtained by multiplying the weight percentage of each monomer with a value obtained by squaring the solubility parameter of the monomer. Specifically, the solubility parameter is determined by the following equation (2).

$$\delta_A = [\phi_1 \times \delta_1^2 + \phi_2 \times \delta_2^2 + \cdots + \phi_n \times \delta_n^2)/100]^{1/2} \quad (2)$$

(wherein $\delta_A$ is the solubility parameter of the acrylic adhesive, $\phi_n$ is the weight percentage (% by weight) of n-th monomer in all the monomers, $\delta_n$ is the solubility parameter of the n-th monomer, and n is an integer representing the number of kinds of monomers.)

Examples of the solid resin include an aminoalkyl (meth)acrylate copolymer, a styrene-based block copolymer, a cellulose derivative, and an alicyclic hydrocarbon resin. The solid resin may be used alone, or two or more kinds thereof may be used in combination.

Examples of the aminoalkyl (meth)acrylate copolymer include copolymers of ethyl acrylate, methyl methacrylate, and trimethyl ammonium ethyl methacrylate chloride (for example, trade name "EUDRAGIT (registered trademark) RS" series, available from BASF), and copolymers of butyl methacrylate, methyl methacrylate, and dimethylaminoethyl methacrylate (for example, trade name "EUDRAGIT (registered trademark) E" series, available from BASF).

Examples of the styrene-based block copolymer include a styrene-butadiene block copolymer, a styrene-butadiene-styrene block copolymer, a styrene-isoprene block copolymer, a styrene-isoprene-styrene block copolymer, a styrene-ethylene/butylene block copolymer, a styrene-ethylene/butylene-styrene block copolymer, a styrene-ethylene/propylene block copolymer, a styrene-ethylene/propylene-styrene block copolymer, a styrene-isobutylene block copolymer, and a styrene-isobutylene-styrene block copolymer. A styrene-isoprene-styrene block copolymer is preferable.

Examples of the cellulose derivative include ethylcellulose, hydroxypropyl cellulose, hydroxypropylmethyl cellulose, hydroxyethyl cellulose, hydroxypropylmethyl cellulose phthalate, and carboxymethyl cellulose. Ethyl cellulose is preferable.

The alicyclic hydrocarbon resin includes an alicyclic saturated hydrocarbon resin and an alicyclic unsaturated hydrocarbon resin.

Examples of the alicyclic saturated hydrocarbon resin include a hydrogenated terpene resin (CLEARON (registered trademark) P, M, and K series available from Yasuhara Chemical Co., Ltd.), hydrogenated rosin and hydrogenated rosin ester-based resins (PENSEL (registered trademark) A available from Arakawa Chemical Industries, Ltd., ESTER GUM (registered trademark) H and HP available from Arakawa Chemical Industries, Ltd., etc.), a hydrogenated terpene phenolic resin (YS POLYSTER UH available from Yasuhara Chemical Co., Ltd., etc.), an aromatic modified hydrogenated terpene resin (CLEARON M and K available from Yasuhara Chemical Co., Ltd.), a hydrogenated dicyclopentadiene-based resin that is a hydrogenated resin of a C5-based petroleum resin obtained by copolymerization of C5 fraction produced by thermal decomposition of petroleum naphtha, such as pentene, isoprene, piperine, and 1,3-pentadiene (Escorez (registered trademark) 5300 and 5400 series available from Exxon Mobil Corporation, and Eastotac (registered trademark) H series available from Eastman Chemical Japan Ltd.), a partially hydrogenated aromatic modified dicyclopentadiene-based resin (Escorez (registered trademark) 5600 series available from Exxon Mobil Corporation), a hydrogenated resin of a C9-based petroleum resin obtained by copolymerization of C9 fraction produced by thermal decomposition of petroleum naphtha, such as indene, vinyltoluene, and $\alpha$- or $\beta$-methylstyrene (ARKON (registered trademark) P and M series available from Arakawa Chemical Industries, Ltd.), and a hydrogenated resin of copolymerized petroleum resin of the C5 and C9 fractions (I-MARV (registered trademark) series available from Idemitsu Kosan Co., Ltd.). A hydrogenated resin of C9-based petroleum resin is preferable.

The alicyclic unsaturated hydrocarbon resin means an alicyclic hydrocarbon resin having an unsaturated bond. Examples of the alicyclic unsaturated hydrocarbon resin include a terpene resin obtained by copolymerization of cyclic terpene such as α-pinene, β-pinene, camphor, and dipentene (unhydrogenated, YS resin (registered trademark) PX and PXN series available from Yasuhara Chemical Co., Ltd., Piccolyte (registered trademark) available from Pinova, Inc.), a terpene phenolic resin (unhydrogenated, YS POLYSTER (registered trademark) U, T, S, G, N, and K series available from Yasuhara Chemical Co., Ltd., etc.), an aromatic modified terpene resin (unhydrogenated, YS resin TO and TR available from Yasuhara Chemical Co., Ltd., etc.), and a petroleum resin obtained by polymerization of 1,3-pentadiene extracted from a C5 fraction produced by thermal decomposition of petroleum naphtha (Neopolymer (registered trademark) available from JX Nippon Oil & Energy Corporation, etc.). A terpene resin and a terpene phenolic resin are preferable.

As the solid resin, the styrene-based block copolymer, the cellulose derivative, and the alicyclic hydrocarbon resin are preferable; a styrene-isoprene-styrene block copolymer, ethyl cellulose, a hydrogenated resin of C9-based petroleum resin, a terpene resin, and a terpene phenolic resin are more preferable; and a styrene-isoprene-styrene block copolymer, ethyl cellulose, and a terpene resin are particularly preferable. These solid resins can impart excellent cohesive force to the adhesive layer while a reduction in tackiness is suppressed.

The content of the solid resin in the adhesive layer is preferably 1 to 25 parts by weight, more preferably 1 to 20 parts by weight, particularly preferably 5 to 20 parts by weight, and the most preferably 7 to 20 parts by weight, relative to 100 parts by weight of the acrylic adhesive. The solid resin contained in a smaller amount may impart insufficient cohesive force to the adhesive layer. The solid resin contained in a larger amount may reduce the tackiness of the adhesive layer.

(Plasticizer)

The adhesive layer contains the plasticizer. It is preferable that the plasticizer be compatible with both the acrylic adhesive and the solid resin.

Examples of the plasticizer include esters, such as isopropyl myristate, decyl oleate, and isopropyl adipate; monohydric alcohols, such as myristyl alcohol, cetanol, octyldodecanol, isostearyl alcohol, and stearyl alcohol; dihydric alcohols, such as octanediol; acids, such as oleic acid and stearic acid; and a liquid paraffin. Among these, isopropyl myristate, octyldodecanol, and a liquid paraffin are preferable, and isopropyl myristate, 2-octyldodecanol, and a liquid paraffin are more preferable. The plasticizer may be used alone, or two or more kinds thereof may be used in combination.

It is preferable that the plasticizer be selected and used according to combination with the acrylic adhesive and the solid resin. When ethyl cellulose and/or a styrene-isoprene-styrene block copolymer is used as the solid resin, it is preferable that the plasticizer contain at least octyldodecanol, it is more preferable that the plasticizer contain a combination of octyldodecanol and isopropyl myristate or a liquid paraffin, and it is particularly preferable that the plasticizer contain a combination of 2-octyldodecanol and a liquid paraffin.

The content of the plasticizer in the adhesive layer is preferably 5 to 80 parts by weight, more preferably 10 to 80 parts by weight, particularly preferably 20 to 70 parts by weight, and the most preferably 20 to 35 parts by weight, relative to 100 parts by weight of the acrylic adhesive. The plasticizer contained in a smaller amount may reduce the tackiness of the adhesive layer. The plasticizer contained in a larger amount may be exuded from the adhesive layer, or may cause the cold flow of the adhesive layer.

(Drug)

The adhesive layer contains the drug. The drug is not limited to a particular one as long as it is capable of transdermal absorption. Examples thereof include Alzheimer drugs, such as donepezil, rivastigmine, galantamine, tacrine, and memantine; antiparkinsonism drugs, such as selegiline and rotigotine; attention-deficit hyperactivity disorder drugs, such as methylphenidate; and anti-inflammatory drugs, such as diclofenac, indometacin, and ethyl salicylate.

It is preferable that the drug be a liquid drug that is in a liquid state at 30°C and has a plasticization effect. Examples of the liquid drug include rivastigmine, selegiline, and ethyl salicylate. These liquid drugs can plasticize the acrylic adhesive, and further improve the anchoring properties of the adhesive layer to the skin surface. When the adhesive patch is attached, the adhesive layer can exert excellent tackiness to the skin. Herein, the liquid state means a state of liquid or a state of fluid.

The content of the drug in the adhesive layer is preferably 1 to 40 parts by weight, more preferably 1 to 30 parts by weight, and particularly preferably 5 to 25 parts by weight, relative to 100 parts by weight of the acrylic adhesive. The drug contained in a smaller amount may not increase the drug blood level to a predetermined range. An excessive amount of the drug contained in a larger amount may be wasteful.

In the adhesive layer, the ratio by weight of the total amount of the plasticizer ($P_1$) and the liquid drug having a plasticization effect ($P_2$) to the amount of the solid resin (S) $[(P_1 + P_2)/S]$ is preferably 1.5 to 25, more preferably 2 to 25, particularly preferably 2.5 to 15, and the most preferably 2.5 to 10. The lower ratio by weight $[(P_1 + P_2)/S]$ may reduce the tackiness of the adhesive layer, or reduce the adhesive force of the adhesive layer with transdermal absorption of the liquid drug having a plasticization effect. The higher ratio by weight $[(P_1 + P_2)/S]$ may impart insufficient cohesive force to the adhesive layer.

(Other Additives)

In addition to the acrylic adhesive, solid resin, and plasticizer described above, the adhesive layer may contain another additive. Examples of the other additive include an absorption promoter, a stabilizer, and a filler.

The filler is used to adjust the shape retentivity of the adhesive layer. Examples of the filler include inorganic fillers, such as silicic anhydride, titanium oxide, and zinc oxide; organic metal salts, such as calcium carbonate and magnesium stearate; and cellulose derivatives, such as lactose, crystalline cellulose, ethyl cellulose, and low-substituted hydroxypropyl cellulose.

The content of the filler in the adhesive layer is preferably 15 parts by weight or less, and more preferably 1 to 10 parts by weight, relative to 100 parts by weight of the acrylic adhesive. The filler contained in a large amount may reduce the tackiness of the adhesive layer.

The thickness of the adhesive layer is not particularly limited, and is preferably 10 to 250 $\mu$m, more preferably 20 to 200 $\mu$m, and particularly preferably 40 to 150 $\mu$m. The thinner adhesive layer cannot contain a sufficient amount of the drug. The thicker adhesive layer may reduce the cohesive force.

[Support]

In the adhesive patch of the present invention, the aforementioned adhesive layer is integrally layered on one surface of the support. The support is required to prevent loss of the drug in the adhesive layer and have strength enough to impart self-supportablity to the adhesive patch. Examples of the support include a resin film, a non-woven fabric, a woven fabric, a knitted fabric, and an aluminum sheet.

Examples of a resin constituting the resin film include cellulose acetate, rayon, polyethylene terephthalate, a plasticized vinyl acetate-vinyl chloride copolymer, nylon, an ethylene-vinyl acetate copolymer, plasticized polyvinyl chloride, polyurethane, polyethylene, polypropylene, and polyvinylidene chloride. Polyethylene terephthalate is particularly preferable since loss of a volatile drug from the adhesive layer is also prevented.

Examples of a material constituting the non-woven fabric include polyethylene, polypropylene, an ethylene-vinyl acetate copolymer, an ethylene-methyl (meth)acrylate copolymer, nylon, a polyester, vinylon, a styrene-isoprene-styrene block copolymer, a styrene-ethylene/butylene-styrene block copolymer, rayon, and cotton. A polyester is preferable. The material may be used alone, or two or more kinds thereof may be used in combination.

The support may be a single layer or a layered sheet in which a plurality of layers are integrally layered. Examples of the layered sheet include layered sheets obtained by integrally layering a polyethylene terephthalate sheet, and either a non-woven fabric or a soft resin sheet.

The thickness of the support is not particularly limited, and is preferably 2 to 200 $\mu$m, and more preferably 2 to 100 $\mu$m.

[Release Liner]

In the adhesive patch of the present invention, a release liner may be integrally and releasably layered on one surface of the adhesive layer. The release liner is used to prevent loss of the drug in the adhesive layer and to protect the adhesive layer.

Examples of the release liner include a paper and a resin film. Examples of a resin constituting the resin film include polyethylene terephthalate, polyethylene, polypropylene, polyvinyl chloride, and polyvinylidene chloride. It is preferable that the release liner be subjected to a release treatment on a surface facing the adhesive layer.

[Method for Producing Adhesive Patch]

Examples of a method for producing the adhesive patch of the present invention include (1) a method in which an adhesive layer solution containing the acrylic adhesive, the solid resin, the plasticizer, the drug, and a solvent is applied to one surface of the support and dried to integrally layer the adhesive layer on the surface of the support, and if necessary, the release liner is layered on the adhesive layer so that a surface that is subjected to a release treatment of the release liner faces the adhesive layer, and (2) a method in which the adhesive layer solution is applied to the surface that is subjected to a release treatment of the release liner and dried to form the adhesive layer on the release liner, and the support is integrally layered on the adhesive layer.

The adhesive layer solution is obtained by uniformly stirring the acrylic adhesive, the solid resin, the plasticizer, the drug, and the solvent. The solvent is not limited to a particular one as long as it allows the acrylic adhesive and the solid resin to be dissolved. For example, toluene, n-hexane, cyclohexane, n-heptane, and ethyl acetate are preferable.

Advantageous Effects of the Invention

The present invention can provide an adhesive patch capable of exerting excellent tackiness when the adhesive patch is attached to the skin and having a low incidence of detachment from the skin during attachment.

Description of Embodiments

Hereinafter, the present invention will be described more specifically by Examples, but the invention is not limited thereto.

Examples

[Preparation of Acrylic Adhesive A]

An acrylic adhesive A was prepared in the following manner. A monomer containing 13 parts by weight of dodecyl methacrylate, 78 parts by weight of 2-ethylhexyl methacrylate, and 9 parts by weight of 2-ethylhexyl acrylate was added to 50 parts by weight of ethyl acetate and stirred to obtain a reaction liquid. Subsequently, the reaction liquid was supplied to a 40-L polymerization device, and the inside of the polymerization device was set to a nitrogen atmosphere of 80°C. Further, 0.5 parts by weight of benzoyl peroxide was dissolved in 50 parts by weight of cyclohexane, to obtain a polymerization initiator solution. While the polymerization initiator solution was added to the reaction liquid over 24 hours, the monomer was copolymerized. After completion of the copolymerization, ethyl acetate was further added to the reaction liquid, and a solution in which the content of the acrylic adhesive A was 35% by weight was obtained. The solubility parameter of the acrylic adhesive A was 9.0 $(cal/cm^3)^{1/2}$.

[Examples 1 to 21 and Comparative Examples 1 and 2]

An acrylic adhesive A, ethyl cellulose (solubility parameter: 10.3 $(cal/cm^3)^{1/2}$, trade name "ETHOCEL" available from DOW Chemical Company), a styrene-isoprene-styrene (SIS) block copolymer (solubility parameter: 8.2 $(cal/cm^3)^{1/2}$, trade name "D1117P" available from Kraton Corporation), a terpene resin (solubility parameter: 8.3 $(cal/cm^3)^{1/2}$, trade name "YS RESIN PX1150N" available from Yasuhara Chemical Co., Ltd.), a hydrogenated resin of C9-based petroleum resin (solubility parameter: 8.2 $(cal/cm^3)^{1/2}$, trade name "ARKON P-100" available from Arakawa Chemical Industries, Ltd.), a terpene phenolic resin (solubility parameter: 8.8 $(cal/cm^3)^{1/2}$, trade name "YS POLYSTER T115" available from Yasuhara Chemical Co., Ltd.), a liquid paraffin (trade name "HICALL M-72" available from Kaneda Co., Ltd.), 2-octyldodecanol, isopropyl myristate, caprylic/capric triglyceride (trade name "Triester F-810" available from Nikko Chemicals Co., Ltd.), and rivastigmine were mixed in respective amounts each shown in Tables 1 to 3, to obtain a mixture. Ethyl acetate and toluene were added to the mixture so that the concentration of the solid content was 25% by weight, and then mixed until the mixture was uniform to produce an adhesive layer solution. Herein, caprylic/capric triglyceride is a triester of glycerine, and caprylic acid and capric acid.

Next, as a release liner, a polyethylene terephthalate film (thickness: 38 μm) that had been subjected to a silicone release treatment was prepared. The adhesive layer solution was applied to the silicone release-treated surface of the release liner and dried at 80°C for 15 minutes to form a layered body in which the adhesive layer (thickness: 100 μm) was formed on the silicone release-treated surface of the release liner.

As a support, a polyethylene terephthalate film (thickness: 25 μm) was prepared. The support and the layered body were layered so that the support and the adhesive layer were faced to each other, and the adhesive layer of the layered body was integrally layered on one surface of the support, to obtain an adhesive patch.

[Evaluation]

For the adhesive patch obtained in each of Examples and Comparative Examples, a ball tackiness test, a constant load peeling test, a 180° peeling test, and a patch test were performed in the following manner. The obtained results were shown in Tables 1 to 3.

(Ball Tack Test (Tackiness))

The adhesive patch was cut at an optional part, to obtain three specimens (22.5 mm in width × 15 mm in length). For each specimen, a maximum ball number was measured in accordance with an inclined ball tack test specified in JIS Z0237 (2009). The arithmetic average of maximum ball numbers of the three specimens was shown in Tables 1 to 3.

(Constant Load Peeling Test (Cohesive Force))

The adhesive patch was cut at an optional part, to obtain a rectangular specimen (20 mm in width × 30 mm in length). Part of the release liner was peeled from the specimen so that a 5-mm terminal of the adhesive layer in the longitudinal direction was exposed. A PET film larger than the exposed part of the adhesive layer was attached to the exposed part of the adhesive layer, to form a clip gripping part. The residual release liner was separated from the specimen, and the specimen was attached to a central part of a rectangular test plate having a test surface formed of an ethylene-vinyl acetate copolymer (EVA) (25 mm in width × 200 mm in length) using the exposed adhesive layer. An 800-g rubber roller was moved back and forth on the specimen one time, and the specimen was allowed to stand at 40°C for 30 minutes, to obtain a layered body. The layered body was stretched horizontally in a constant temperature bath of 40°C with the specimen facing downward. After that, a 1-g metal clip was attached to the clip gripping part, and a 1-g weight was hung on the metal clip to initiate the test. 24 hours after initiation of the test, the longest peeling length (mm) of the specimen peeled from the test plate was measured. From the optional part of the adhesive patch, three specimens were prepared, and the longest peeling lengths of the three specimens were each measured in the aforementioned manner. The arithmetic average thereof was shown in Tables 1 to 3.

(180° Peeling Test (Adhesive force))

The adhesive patch was cut at an optional part, to obtain three specimens (22.5 mm in width × 15 mm in length). For each specimen, a peeling adhesive force (g/25 mm) was measured in accordance with 180° peeling test specified in JIS Z0237 (2009). The arithmetic average thereof was shown in Tables 1 to 3.

(Patch Test)

The adhesive patch was cut at an optional part, to obtain ten specimens (area: 10 cm$^2$). From each specimen, the release liner was separated, and each specimen was attached to upper arm parts of ten adult healthy men. 24 hours after the attachment, the area of the specimen attached to the skin was measured. The attachment property was evaluated in accordance with the following adhesion scores. The arithmetic average of adhesion score of each specimen was shown in Tables 1 to 3.

<Adhesion Score>

0: the adhesion area was 90% or more.
1: the adhesion area was 75% or more and less than 90%.
2: the adhesion area was 50% or more and less than 75%.
3: the adhesion area was less than 50%, but the specimen was not detached.
4: the specimen was detached from the skin.

[Table 1]

| FORMULATION (PART BY WEIGHT) | | | EXAMPLE | | | | | | | | |
|---|---|---|---|---|---|---|---|---|---|---|---|
| | | | 1 | 2 | 3 | 4 | 5 | 6 | 7 | 8 | 9 |
| FORMULATION (PART BY WEIGHT) | ACRYLIC ADHESIVE A | | 100 | 100 | 100 | 100 | 100 | 100 | 100 | 100 | 100 |
| | SOLID RESIN (S) | ETHYL CELLULOSE | 16 | 16 | 7 | 17 | 16 | 15 | 17 | 0 | 0 |
| | | SIS BLOCK COPOLYMER | 0 | 0 | 0 | 0 | 0 | 0 | 0 | 3 | 6 |
| | PLASTICIZER ($P_1$) | LIQUID PARAFFIN | 0 | 8 | 7 | 15 | 16 | 0 | 0 | 7 | 7 |
| | | 2-OCTYLDODECANOL | 20 | 14 | 13 | 15 | 6 | 13 | 15 | 13 | 13 |
| | | ISOPROPYL MYRISTATE | 0 | 0 | 0 | 0 | 0 | 7 | 15 | 0 | 0 |
| | DRUG ($P_2$) | RIVASTIGMINE | 20 | 21 | 19 | 22 | 21 | 19 | 22 | 18 | 18 |
| | | RATIO BY WEIGHT [$(P_1+P_2)$/S] | 2.6 | 2.7 | 5.4 | 3.1 | 2.7 | 2.7 | 3.1 | 13.5 | 6.8 |
| EVALUATION | BALL TACK TEST | MAXIMUM BALL NUMBER | 9 | 10 | 10 | 10 | 9 | 9 | 10 | 10 | 11 |
| | CONSTANT LOAD PEELING TEST | LONGEST PEELING LENGTH (mm) | 0.6 | 1.3 | 1.0 | 1.0 | 1.0 | 1.0 | 1.0 | 2.7 | 2.0 |
| | 180° PEELING TEST | PEELING ADHESIVE FORCE (mg/25mm) | 181 | 198 | 177 | 217 | 235 | 213 | 247 | 187 | 186 |
| | PATCH TEST | ADHESION SCORE | 0 | 0 | 0 | 0 | 0 | 0 | 0 | 0 | 0 |

[Table 2]

| FORMULATION (PART BY WEIGHT) | | | EXAMPLE | | | | | | | COMPARATIVE EXAMPLE | |
|---|---|---|---|---|---|---|---|---|---|---|---|
| | | | 10 | 11 | 12 | 13 | 14 | 15 | 16 | 1 | 2 |
| FORMULATION (PART BY WEIGHT) | ACRYLIC ADHESIVE A | | 100 | 100 | 100 | 100 | 100 | 100 | 100 | 100 | 100 |
| | SOLID RESIN (S) | ETHYL CELLULOSE | 0 | 15 | 24 | 7 | 14 | 15 | 15 | 0 | 0 |
| | | SIS BLOCK COPOLYMER | 3 | 0 | 0 | 0 | 0 | 0 | 0 | 0 | 0 |
| | PLASTICIZER ($P_1$) | LIQUID PARAFFIN | 0 | 0 | 0 | 0 | 0 | 13 | 0 | 0 | 0 |
| | | 2-OCTYLDODECANOL | 20 | 13 | 14 | 12 | 7 | 0 | 0 | 13 | 13 |
| | | ISOPROPYL MYRISTATE | 0 | 0 | 0 | 0 | 0 | 0 | 13 | 0 | 0 |
| | DRUG ($P_2$) | RIVASTIGMINE | 18 | 19 | 21 | 18 | 18 | 19 | 19 | 0 | 18 |
| | | RATIO BY WEIGHT [($P_1$+$P_2$)/S] | 13.5 | 2.2 | 1.5 | 4.4 | 1.8 | 2.2 | 2.2 | - | - |
| EVALUATION | BALL TACK TEST | MAXIMUM BALL NUMBER | 10 | 8 | 7 | 10 | 5 | 10 | 8 | 3 | 13 |
| | CONSTANT LOAD PEELING TEST | LONGEST PEELING LENGTH (mm) | 3.0 | 2.3 | 4.8 | 3.0 | 3.5 | 3.3 | 7.4 | 25 | 25 |
| | 180° PEELING TEST | PEELING ADHESIVE FORCE (mg/25mm) | 199 | 178 | 164 | 154 | 176 | 334 | 464 | 873 | 362 |
| | PATCH TEST | ADHESION SCOPE | 1 | 1 | 1 | 1 | 1 | 1 | 1 | 2 | 2 |

EP 3 251 663 A1

[Table 3]

| | | | EXAMPLE | | | | |
|---|---|---|---|---|---|---|---|
| | | | 17 | 18 | 19 | 20 | 21 |
| FORMULATION (PART BY WEIGHT) | ACRYLIC ADHESIVE A | | 100 | 100 | 100 | 100 | 100 |
| | SOLID RESIN (S) | ETHYL CELLULOSE | 0 | 0 | 0 | 22 | 0 |
| | | SIS BLOCK COPOLYMER | 0 | 0 | 0 | 0 | 0 |
| | | TERPENE RESIN | 16 | 0 | 25 | 0 | 0 |
| | | HYDROGENATED RESIN OF C9-BASED PETROLEUM RESIN | 0 | 15 | 0 | 0 | 22 |
| | | TERPENE PHENOLIC RESIN | 0 | 0 | 0 | 22 | 0 |
| | PLASTICIZER ($P_1$) | LIQUID PARAFFIN | 24 | 0 | 20 | 31 | 0 |
| | | 2-OCTYLDODECANOL | 0 | 13 | 0 | 0 | 6 |
| | | ISOPROPYL MYRISTATE | 0 | 0 | 0 | 11 | 0 |
| | | CAPRYLIC/CAPRIC TRIGLYCERIDE | 0 | 0 | 0 | 11 | 0 |
| | DRUG ($P_2$) | RIVASTIGMINE | 21 | 19 | 22 | 24 | 19 |
| | | RATIO BY WEIGHT [$(P_1+P_2)$/S] | 2.8 | 2.2 | 1.7 | 1.8 | 1.1 |
| EVALUATION | BALL TACK TEST | MAXIMUM BALL NUMBER | 12 | 14 | 5 | 5 | 10 |
| | CONSTANT LOAD PEELING TEST | LONGEST PEELING LENGTH (mm) | 1.8 | 3.7 | 4.4 | 15.1 | 20.2 |
| | 180° PEELING TEST | PEELING ADHESIVE FORCE (mg/25mm) | 307 | 572 | 371 | 618 | 512 |
| | PATCH TEST | ADHESION SCORE | 0 | 1 | 1 | 2 | 2 |

(Cross-reference to Related Applications)

This application claims priority on the basis of Japanese Patient Application No. 2015-16991, filed on January 30, 2015, the disclosure of which is incorporated herein by reference in its entirety.

Industrial Applicability

The present invention can provide an adhesive patch capable of exerting excellent tackiness to the skin when the adhesive patch is attached and having a low incidence of detachment from the skin during attachment. According to the adhesive patch of the present invention, a drug can be transdermally administered with stability.

**Claims**

1.  An adhesive patch comprising:

    a support; and
    an adhesive layer integrally layered on one surface of the support, the adhesive layer containing an acrylic adhesive, a solid resin that is incompatible with the acrylic adhesive and is in a solid state at 40°C, a plasticizer, and a drug.

**2.** The adhesive patch according to claim 1, wherein a difference between a solubility parameter of the acrylic adhesive and a solubility parameter of the solid resin is 0.5 $(cal/cm^3)^{1/2}$ or more.

**3.** The adhesive patch according to claim 1 or 2, wherein the solid resin contains at least one kind of resin selected from the group consisting of ethyl cellulose, a styrene-isoprene-styrene block copolymer, and a terpene resin.

**4.** The adhesive patch according to any one of claims 1 to 3, wherein the adhesive layer contains 1 to 25 parts by weight of the solid resin relative to 100 parts by weight of the acrylic adhesive.

**5.** The adhesive patch according to any one of claims 1 to 4, wherein the plasticizer contains at least one kind selected from the group consisting of octyldodecanol, isopropyl myristate, and a liquid paraffin.

**6.** The adhesive patch according to any one of claims 1 to 5, wherein the adhesive layer contains 5 to 80 parts by weight of the plasticizer relative to 100 parts by weight of the acrylic adhesive.

**7.** The adhesive patch according to any one of claims 1 to 6, wherein the drug contains a liquid drug that is in a liquid state at 30°C and has a plasticization effect.

**8.** The adhesive patch according to any one of claims 1 to 7, wherein the drug contains at least one kind of liquid drug having a plasticization effect selected from the group consisting of rivastigmine, selegiline, and ethyl salicylate.

**9.** The adhesive patch according to claim 7 or 8, wherein a ratio by weight of a total amount of the plasticizer ($P_1$) and the liquid drug having a plasticization effect ($P_2$) to an amount of the solid resin (S) $[(P_1 + P_2)/S]$ is 1.5 to 25.

# EP 3 251 663 A1

## INTERNATIONAL SEARCH REPORT

| | International application No. |
|---|---|
| | PCT/JP2016/052294 |

### A. CLASSIFICATION OF SUBJECT MATTER

*A61K9/70*(2006.01)i, *A61K31/137*(2006.01)i, *A61K31/27*(2006.01)i, *A61K31/618*(2006.01)i, *A61K47/06*(2006.01)i, *A61K47/10*(2006.01)i, *A61K47/14*(2006.01)i, *A61K47/32*(2006.01)i, *A61K47/38*(2006.01)i, *A61P17/00*(2006.01)i,

According to International Patent Classification (IPC) or to both national classification and IPC

### B. FIELDS SEARCHED

Minimum documentation searched (classification system followed by classification symbols)
A61K9/70, A61K31/137, A61K31/27, A61K31/618, A61K47/06, A61K47/10, A61K47/14, A61K47/32, A61K47/38, A61P17/00, A61P25/16, A61P25/28

Documentation searched other than minimum documentation to the extent that such documents are included in the fields searched
| Jitsuyo Shinan Koho | 1922–1996 | Jitsuyo Shinan Toroku Koho | 1996–2016 |
| Kokai Jitsuyo Shinan Koho | 1971–2016 | Toroku Jitsuyo Shinan Koho | 1994–2016 |

Electronic data base consulted during the international search (name of data base and, where practicable, search terms used)
WPI, JSTPlus/JMEDPlus/JST7580(JDreamIII)

### C. DOCUMENTS CONSIDERED TO BE RELEVANT

| Category* | Citation of document, with indication, where appropriate, of the relevant passages | Relevant to claim No. |
|---|---|---|
| X<br>Y | WO 02/069942 A1 (Hisamitsu Pharmaceutical Co., Inc.),<br>12 September 2002 (12.09.2002),<br>claims 1, 4; page 8, line 2 to page 10, line 24<br>& JP 4261911 B      & US 2004/0096491 A1<br>claims 1, 4; paragraph [0035]<br>& EP 1366762 A1      & BR 207955 A<br>& KR 10-0846642 B1    & CN 1499962 A | 1-9<br>1-6 |

☒ Further documents are listed in the continuation of Box C.    ☐ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 28 March 2016 (28.03.16) | 05 April 2016 (05.04.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

| International application No. |
| --- |
| PCT/JP2016/052294 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y | JP 2014-125466 A (Hisamitsu Pharmaceutical Co.,<br>Inc.),<br>07 July 2014 (07.07.2014),<br>claims 1, 6; paragraph [0039]; examples<br>& US 2014/0186428 A1<br>claims 1, 6; paragraph [0070]; examples<br>& EP 2749274 A1 & TW 201347794 A<br>& CA 2820479 A & KR 10-1392064 B<br>& CN 103893156 A & ES 2554115 T | 1-9<br>1-6 |
| X<br>Y | JP 2004-083523 A (Hisamitsu Pharmaceutical Co.,<br>Inc.),<br>18 March 2004 (18.03.2004),<br>claims; paragraphs [0039], [0046], [0048],<br>[0049]; examples<br>& US 2005/0260255 A1<br>claims; paragraphs [0042], [0049], [0051],<br>[0052]; examples<br>& WO 2004/019988 A1 & EP 1541177 A1<br>& KR 10-2005-0057053 A & CN 1678351 A<br>& AU 2003254875 A | 1-9<br>1-6 |
| X<br>Y | JP 07-101852 A (Sekisui Chemical Co., Ltd.),<br>18 April 1995 (18.04.1995),<br>claim 1; examples<br>(Family: none) | 1-9<br>1-6 |
| X<br>Y | JP 09-301854 A (Sekisui Chemical Co., Ltd.),<br>25 November 1997 (25.11.1997),<br>claim 1; paragraphs [0026], [0027]; example 2<br>(Family: none) | 1-6<br>1-9 |
| X<br>Y | WO 2008/032719 A1 (Sekisui Chemical Co., Ltd.),<br>20 March 2008 (20.03.2008),<br>claims; paragraphs [0040], [0043]; example 19<br>& JP 4445568 B & US 2009/0317452 A1<br>claims; paragraphs [0040], [0043]; example 19<br>& EP 2062576 A1 & KR 10-2009-0051191 A<br>& AT 543490 T | 1-6<br>1-9 |
| Y | WO 2014/047191 A1 (MYLAN INC.),<br>27 March 2014 (27.03.2014),<br>claims; table 4<br>& JP 2015-535827 A & US 2014/0083878 A1<br>& EP 2897598 A1 & CA 2884108 A<br>& AU 2013318116 A | 1-9 |
| Y | WO 2014/051128 A1 (KM Transderm Ltd.),<br>03 April 2014 (03.04.2014),<br>particularly, examples<br>& US 2015/0374642 A1<br>especially, examples<br>& EP 2859892 A1 & CN 104114167 A<br>& TW 201521795 A | 7-9 |

Form PCT/ISA/210 (continuation of second sheet) (January 2015)

**INTERNATIONAL SEARCH REPORT**

International application No.

PCT/JP2016/052294

C (Continuation).  DOCUMENTS CONSIDERED TO BE RELEVANT

| Category* | Citation of document, with indication, where appropriate, of the relevant passages | Relevant to claim No. |
|---|---|---|
| Y | WO 2013/047410 A1 (Nichiban Co., Ltd.),<br>04 April 2013 (04.04.2013),<br>claims; examples<br>(Family: none) | 7-9 |

Form PCT/ISA/210 (continuation of second sheet) (January 2015)

**INTERNATIONAL SEARCH REPORT**

International application No.

PCT/JP2016/052294

Continuation of A. CLASSIFICATION OF SUBJECT MATTER
  (International Patent Classification (IPC))

A61P25/16(2006.01)i, A61P25/28(2006.01)i

              (According to International Patent Classification (IPC) or to both national
              classification and IPC)

Form PCT/ISA/210 (extra sheet) (January 2015)

16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4724368 B

- JP 2015016991 A

**Non-patent literature cited in the description**

- Youkaisei Parameter Tekiyo Jirei-syu (Case Studies of Application of Solubility Parameter). Akitoshi Taniguchi, Johokiko Co., Ltd, 15 March 2007, 276-282